# EUROPEAN PATENT APPLICATION

(11) **EP 1 797 785 A1**
(43) Date of publication of application: **20.06.2007**
(21) Application number: 05112405.5
(22) Date of filing: 19.12.2005
(51) Int. Cl.: A43B 17/02, A61B 5/103, A43B 7/00

(54) **Orthopedic insole**

(71) Applicant: CAMP SCANDINAVIA AB, 254 67 Helsingborg (SE)
(72) Inventor: Köhler, Peter, 182 75, Stocksund (SE); Lind, Leif, 184 32, Åkersberga (SE)
(74) Representative: Akerman, Marten Lennart

(57) **Abstract**

The invention relates generally to an orthopedic insole arrangement comprising a bottom insole (110), a top insole (112) and a set of overload tactile feedback elements, e.g. pins (115, 116, 117, 118, 119) forming an orthopedic insole kit. The assembled insole arrangement gives a tactile overload feedback to patients during rehabilitation, such as during walking, allowing an optimised healing process. The insole (913) may comprise a bottom insole (110) having at least one hole (120, 130, 140, 150, 160, 165, 170, 175, 180, 185), and a top insole (112) with matching hole/s (121, 131, 141, 151, 161, 166, 171, 176, 181, 186), wherein at least one pin (115, 116, 117, 118, 119) is installed to slide vertically inside the hole (121, 131, 141, 151, 161, 166, 171, 176, 181, 186), thereby protruding from the insole (913) at some compression of the insole (913).

## Description

### TECHNICAL FIELD

The invention relates to an insole as defined in the preamble of claim 1, and a bottom insole as defined in the preamble of claim 10, a top insole as defined in the preamble of claim 12 and an insole kit as defined in the preamble of claim 14.

### BACKGROUND

The rehabilitation period for healing injuries in the lower extremities is normally several weeks or even months, e.g. after bone fractures, sprains, chirurgical operations, diseases etc. Apart from physical and psychological suffering, and thus a decreased quality of life for the patients, these relatively long rehabilitation periods give rise to substantial costs for society, mainly in form of a fall in production. There is a long felt need to fmd effective and efficient means and methods for shortening these rehabilitation periods. A leg/foot should normally be used to some extent for optimal tissue recovery while a too heavy work load on a leg/foot during rehabilitation may give severe negative, and even catastrophic, consequences for the healing process. There is a problem to find efficient and effective means which allow patients to use and physically stimulate their legs/feet for optimal tissue recovery, and which at the same time guarantee that there is no risk of over-stimulation, i.e. overload, of the injured part/s in question with possible negative/catastrophic consequences for the healing process. Various aids are used today for helping patients to provide optimal load stimulation of a leg/foot during rehabilitation.

The patent document DE 4116124 C1 discloses an overload alarm arrangement to be installed in a heel sole of a shoe. A resilient member is compressed by the foot during walking and thereby exposes a tactile feedback member, which in turn provides a tactile feedback to the patient in case of overload.

The document DE 10038446 A1 discloses a sole arrangement exploiting pressure sensors which provide acoustic/tactile feedback in case of overload. The pressure sensors are arranged in a bottom sole which is covered by a top insole.

The patent document US 5269081 discloses a force monitoring shoe for monitoring the force being applied to a patient's leg, as during walking. The device is contained within a shoe-like enclosure and an alert system notifies the patient when a certain pre-established force value has been reached.

The article "A new ambulatory foot pressure device for patients with sensory impairment. A system for continuous measurement of plantar pressure and a feedback alarm", by Z. Pataky et al, published in the Journal of Biomechanics 33 (2000), page 1135-1138, ISSN:0021-9290, discloses a device for solving problems concerning neuropathic ulcers on feet. The device comprises pressure sensors positioned under a patient's foot which sensors provide an alarm at a certain pressure allowing the patient to lower the work load on the foot when a certain threshold load has been reached.

The patent document US 6273863 discloses an adaptive weight bearing monitoring system for rehabilitation of injuries of the lower extremities, e.g for orthopedic patients, which compare weight forces applied to patient with set input weight range based on which stimulation signal is applied to patients.

The patent document JP 2004141275 A2 discloses a sole pressure-distribution hearing biofeedback system for rehabilitation medical treatment, which system outputs sound corresponding to audio data generated with respect to detected sole pressure of person during walking.

Problems with these state of the art gait correction aids mainly concern an unsatisfactory user friendliness, a too high production cost and/or usage cost and that they are generally not very versatile. Therefore, they are not very effective or efficient.

### SUMMARY OF THE INVENTION

The present invention seeks to mitigate/solve above problems.

It is an object of the present invention to optimize the healing process of injuries in the lower extremities during rehabilitation, thereby increasing the quality of life for the patients concerned and shortening the rehabilitation period for such injuries.

This object is achieved according to a first aspect of the invention by providing a compressible insole comprising at least one hole wherein said insole comprises at least one tactile overload feedback element being anchored in the insole and being arranged to slide inside the hole in a substantially perpendicular direction relative a foot during normal operation when the insole is being compressed at weight bearing, as during walking, thereby protruding from the insole at some compression of the insole (913).

In this way the invention thus provides an orthopedic insole which gives a tactile overload feedback to patients during rehabilitation, such as during walking, whenever said tactile overload feedback element protrudes from the insole at some weight load during walking and contacts the patient's foot, thus providing the tactile overload feedback to the patient. The orthopedic insole of the invention is user friendly, i.e. easy to use both for patients and health care organizations, cost effective and versatile, i.e. applicable for many different patient groups in various environments and conditions, e.g. being applicable for patients in plaster and non in plaster etc.

In one embodiment, said hole is positioned vertically under the forefoot, or the heel of the foot, during normal operation, as during walking. This provides for good tactile feedback characteristics since the hole, and thereby also the tactile overload feedback element, during normal operation is positioned at a relatively high weight load area where the nerve distribution of the foot is also relatively dense.

In one embodiment, said hole is positioned under a first metatarsal head of the foot or under the third metatarsal head of the foot or under the fifth metatarsal head of the foot or under the calcaneus bone of the foot during normal operation, as during walking. This provides for particularly good tactile feedback characteristics of the insole.

In one embodiment, the insole comprises five holes perpendicularly oriented relative the foot, and five tactile overload feedback elements, each tactile overload feedback element being arranged to slide in one of the respective holes during weight bearing, as during walking, wherein two of the holes are positioned under the calcaneus bone, one hole is positioned under the first metatarsal head, one hole is positioned under the third metatarsal head and one hole is positioned under the fifth metatarsal head during normal operation, as during walking. This provides for effective tactile overload feedback to the patient during the entire ground contact phase of the gait cycle when the lower extremities are under stress.

In one embodiment, the insole comprises a bottom insole and a top insole arranged on top of the bottom insole wherein the bottom insole is made of a firm material, such as a thermoplastic polyamide material, and said tactile overload feedback element is anchored in said bottom insole; and wherein said top insole is made of a compressible material, such as a cellular plastic material, a cellular urethane material, a polyether material or a cellular rubber material, and comprises said at least one hole in which the tactile overload feedback element is arranged to slide during normal operation. This provides a cost efficient insole realisation wherein the bottom insole may be re-used by a plurality of patients. Furthermore, separate top/bottom insoles makes it possible to use the insole for both the left and the right foot by simply turning the bottom insole upside down and installing the pins in an opposite direction and then mount the top insole (also reversed upside down) on top of the bottom insole. This also provides a cost efficient solution for updating the tactile feedback characteristics of the insole by simply replacing the top insole of the insole with another top insole having other compression properties.

In one embodiment, the bottom insole comprises at least one through hole matching said at least one through hole of the top insole during normal operation, wherein the at least one tactile overload feedback element is snapped onto the bottom insole through said through hole of the bottom insole. This is a cost efficient solution for which it is possible to use the insole for both the left and the right foot by simply turning the bottom insole upside down and simply snap "off" and "on" the tactile overload feedback element/s from/to the bottom insole in an opposite direction and then mount the top insole (also reversed upside down) on top of the bottom insole.

In one embodiment, the top insole is made of a compressible material having a hardness shore "0" value in the range of A 4-30. This assures that the tactile overload feedback element will slide in said hole and protrude at some stress load during normal operation.

In one embodiment, the insole, when being aligned with a sagittal plane of a foot during normal operation, and further being associated with an imaginary system of coordinates defining a first and second region of the insole; said system of coordinates having an Y-axis being parallel with said sagittal plane and substantially directed in a frontal direction of the insole, and having an X-axis directed in a medial direction, and having its origin of coordinates positioned at the backmost edge end of the insole when so aligned; the X-axis and Y-axis defining coordinates of length in mm; said regions being defined by the following:
- a first set of coordinates fulfilling the criteria of y<117 and having a minimum distance to an outer edge of the insole of 12 mm, wherein the first set of coordinates define the first region of the insole, and,
- a second set of coordinate points being defined by fulfilling the criteria of 157,5<y<273 and having a minimum distance to an outer edge of the insole of 12 mm, wherein the second set of coordinates define the second region of the insole,
wherein the insole have a total length of about 299 mm from the back edge end to a front edge end, a heel region width of about 70 mm and a front foot region width of about 100 mm, and wherein the at least one through hole of the bottom insole and the at least one through hole of the top insole lead to said first region or to said second region of the insole. This allows for the production of one standard size insole adaptable to fit patients with different shoe sizes which standard size insole has good tactile feedback characteristics.

In one embodiment, the top insole and the bottom insole each comprise 10 respective matching through holes, each respective through hole being positioned as follows:
- a first respective through hole positioned at the coordinate point (x=0, y=31),
- a second respective through hole positioned at the coordinate point (x=-0,7, y=46),
- a third respective through hole positioned at the coordinate point (x=-1,2, y=58),
- a fourth respective through hole positioned at the coordinate point (x=-1,7, y=70),
- a fifth respective through hole positioned at the coordinate point (x=-23, y=198),
- a sixth respective through hole positioned at the coordinate point (x=0, y=205),
- a seventh respective through hole positioned at the coordinate point (x=22, y=195),
- an eighth respective through hole positioned at the coordinate point (x=-25, y=210),
- a ninth respective through hole positioned at the coordinate point (x=0, y=220),
- a tenth respective through hole positioned at the coordinate point (x=30, y=208), and wherein five tactile overload feedback elements are being arranged to slide vertically in a respective hole of the top insole during normal operation. This provides for effective tactile overload feedback to the patient during the entire ground contact phase of the gait cycle when the lower extremities are under stress.

In one embodiment, the top insole has a thickness of about 6 mm at rest and, at least in some region, is compressed at least about 1 mm during normal operation, as during walking. This makes the insole not so bulky and provides a possibility to use it in a conventional shoe.

In one embodiment, the top insole resumes its substantial rest thickness of about 6 mm within about 3 seconds after foot pressure release. This assures that the insole will give substantially the same tactile feedback response for a plurality of ground contact phases.

In one embodiment, the bottom insole has a thickness of about 0,5 mm and the diameter of the at least one through hole of the bottom insole is about 4 mm and the diameter of the at least one through hole of the top insole is about 5 mm. This assists in providing a not so bulky insole in which an effective tactile overload feedback of suitable dimensions may easily be installed.

In one embodiment, said tactile overload feedback element is made of a firm material, such as plastic or a metallic material, and is realised as a pin element or a half disk element or a half spherical element. This assures good tactile stimulation to the foot.

According to a second aspect, the invention provides a bottom insole as defined according to the first aspect of the invention.

In on embodiment, the bottom insole has at least one shoe size number contour line printed or otherwise outlined on it which contour line corresponds to a shoe number size in the size number range of 35-47 according to a Swedish shoe size standard. This facilitates a correct size adjustment of a standard size insole to fit patients with different shoe sizes.

According to a third aspect, the invention provides a top insole as defined according to the first aspect of the invention.

In on embodiment, the top insole has at least one shoe size number contour line printed or otherwise outlined on it which contour line corresponds to a shoe number size in the size number range of 35-47 according to a Swedish shoe size standard. This facilitates a correct size adjustment of a standard size insole to fit patients with different shoe sizes.

According to a fourth aspect, the invention provides a bottom insole according to the second aspect of the invention and a top insole according to the third aspect of the invention.

In one embodiment, the kit further comprises a tactile overload feedback element being made of a firm material, such as plastic or a metallic material, and realised as a pin element or a half disk element or a half spherical element, wherein the tactile overload feedback element, when installed in the insole, penetrates about 5 mm into the at least one through hole of the top insole.

Even though the invention has been summarized above, the invention is defined by the accompanying claims 1-19.

### BRIEF DESCRIPTION OF THE DRAWINGS

The features and advantages of the present invention will become more apparent from the following detailed description of the preferred embodiments with reference to the accompanying drawings, wherein;
- FIG 1: illustrates an orthopedic insole kit according to one embodiment of the present invention,
- FIG 2: illustrates a human foot and the hole positioning approach for the insoles of FIG 1, according to one embodiment of the invention,
- FIG 3: illustrates a human foot and its bones along with form indications referring to the foot in FIG 2,
- FIG 4: illustrates a top view of any of the insoles in FIG 1, according to one embodiment of the invention,
- FIG 5: is a top view of an insoleillustrating the positioning of 10 through holes according to one embodiment of the invention,
- FIG 6A: is a side view of a top insole, according to one embodiment of the invention,
- FIG 6B:: is a side view of a bottom insole, according to one embodiment of the invention,
- FIG 7: illustrates a top view of an insole according to one embodiment of the invention.
- FIG 8: illustrates a pin according to one embodiment of the invention,
- FIG 9A: illustrates the insole arrangement according to the invention during usage,
- FIG 9B: illustrates the insole arrangement according to the invention during usage,
- FIG 10A: illustrates a foot-ground contact for a specific patient group,
- FIG 10B: illustrates a foot-ground contact for a specific patient group.

### DETAILED DESCRIPTION OF PREFERRED EMBODIMENTS

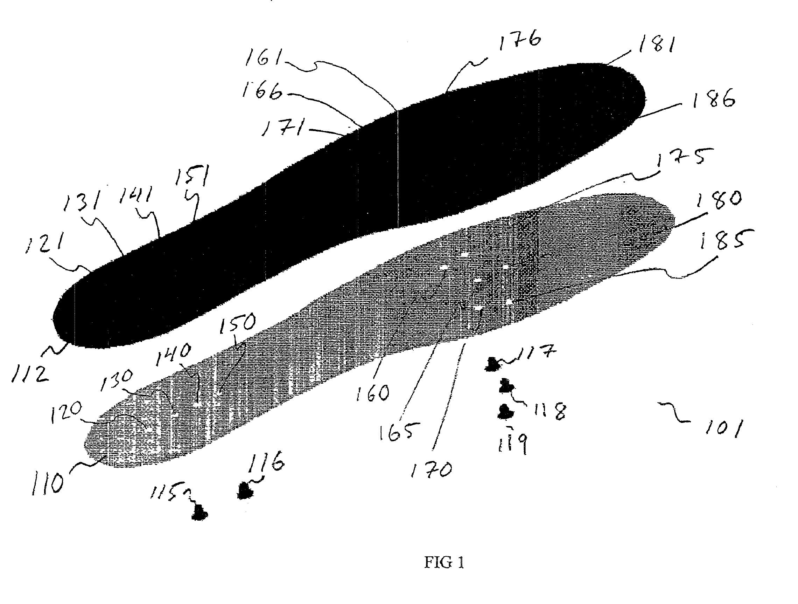

Now, with reference to FIG 1-10, the present invention shall be described in more detail.

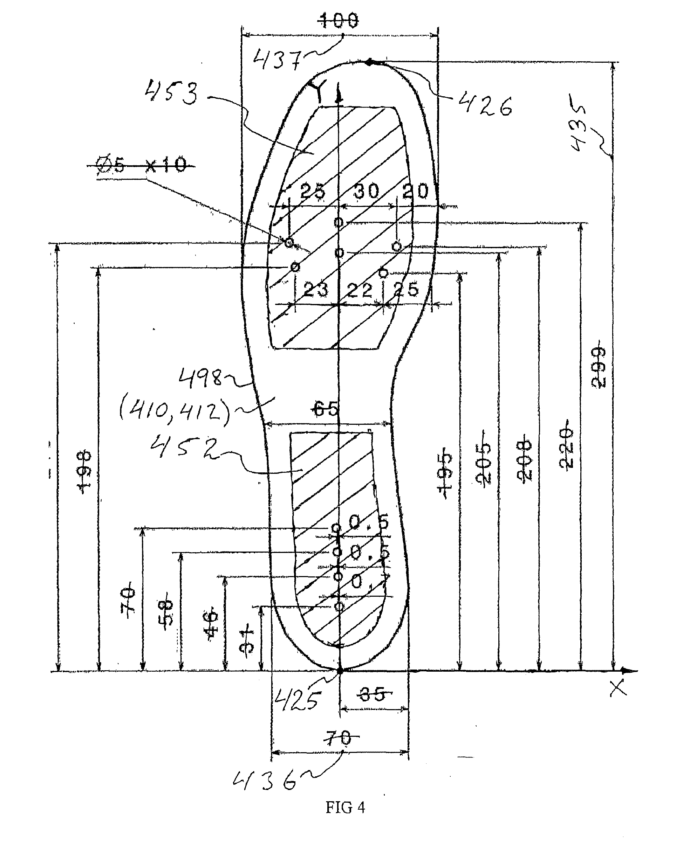

In the Figures 1-10, corresponding elements, or length indications, have been given the same reference number along with a figure prefix number, e.g. the bottom insole 112 in FIG 1 is referred to as insole 412 in FIG 4 etc. The figures are not to scale and the relative dimensions of the illustrated objects may be disproportional.

The invention is based on the concept to provide accurate tactile feedback to patients during walking at specific foot weight loads allowing them to auto-control, and thereby limit, the load on bones, ligaments etc. during walking, thereby optimising the healing/recovery process of injured/traumatised tissue in the lower extremities, e.g. after bone fractures, sprains etc. The tactile feedback is provided by means of an orthopedic insole arrangement.

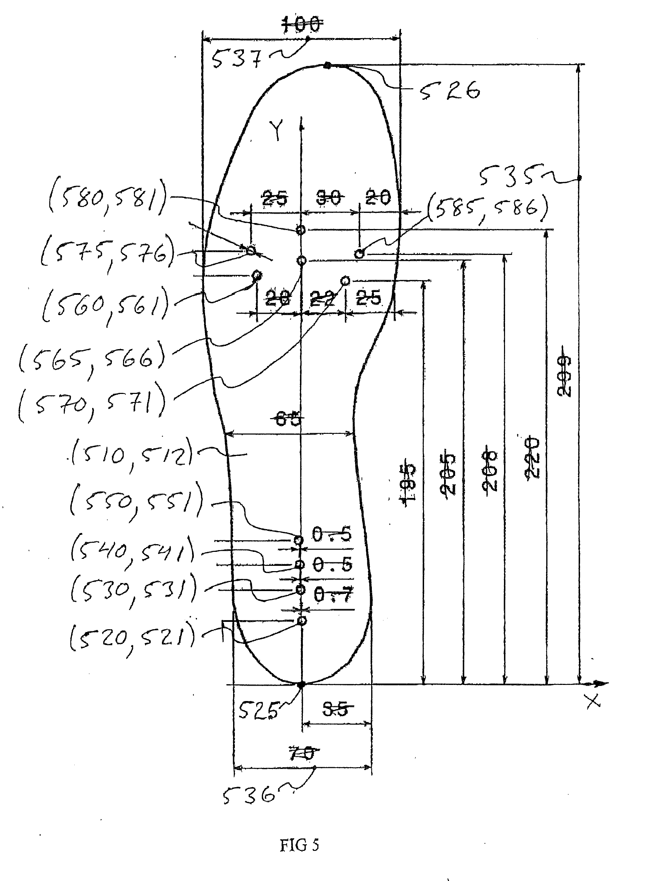

FIG 1 illustrates an orthopedic insole kit 101 according to one embodiment of the present invention, i.e. an unassembled orthopedic insole kit comprising a bottom insole 110, a top insole 112, and 5 tactile overload feedback elements, here realised as pins 115, 116, 117, 118, 119. In Figure 1, 5 pins are shown but any suitable number of pins may be used. According to the invention, the bottom insole 110 and top insole 112 have matching through holes, illustrated by holes 120, 130, 140, 150, 160, 165, 170, 175, 180, 185 of the bottom insole 110 and corresponding holes 121, 131, 141, 151, 161, 166, 171, 176, 181, 186 of the top insole 112, in FIG 1. The pins 115, 116, 117, 118, 119 are then snapped into the holes 120, 130, 140, 150, 160, 165, 170, 175, 180, 185 of the bottom sole 110, and the top insole 112 is mounted on top of the bottom insole 110, so that the pins run in the holes 121, 131, 141, 151, 161, 166, 171, 176, 181, 186 of the top insole 112. The design of the pins is typically such that it allows an easy "snap on" and a firm anchoring in the bottom insole. The holes 120, 121, 130, 131, 140, 141, 150, 151, 160, 161, 165, 166, 170, 171, 175, 176, 180, 181, 185, 186 illustrated in FIG 1 need not be circular, but circular holes may provide advantages e.g. regarding production costs etc. Alternatively, the bottom insole 110 may have integrated protrusions fitting into the matching holes 121, 131, 141, 151, 161, 166, 171, 176, 181, 186 of the top insole. In this way, an assembled orthopaedic insole 113 is created comprising the top insole 112, the bottom insole 110 and the pins 115, 116, 117, 118, 119. As described further below, the pins 115, 116, 117, 118, 119 run in the holes 121, 131, 141, 151, 161, 166, 171, 176, 181, 186 of the top insole 112 and reach the skin of the foot at a certain pressure/compression. If the top insole 112 is stressed further, the pins then provide a distinct tactile feedback to the patient, at a certain specific weight load.

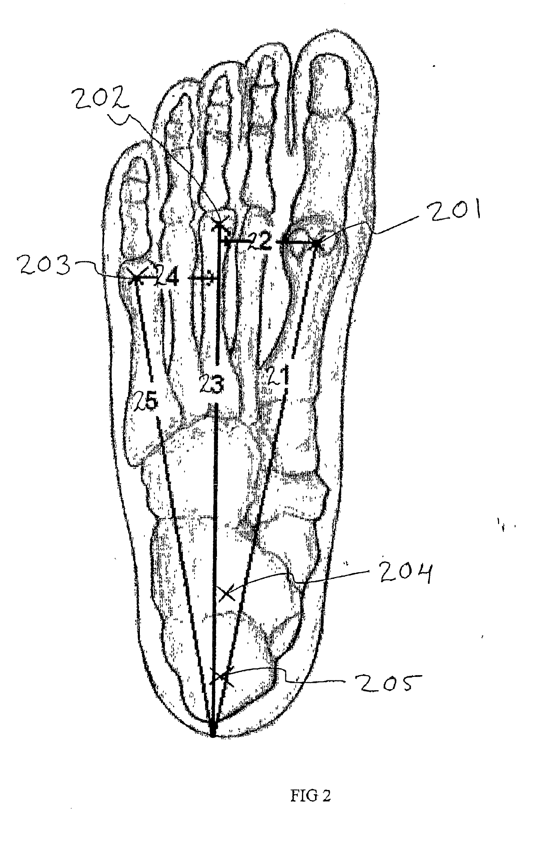

FIG 2 illustrates where the holes, and pins, are to be positioned for optimal performance, according to one embodiment of the invention. Three pins are positioned at the respective head of the 1:st, 3:rd and 5:th metatarsal bone, illustrated by points 201, 202 and 203 in FIG 2, and corresponding to holes 160, 161, 165, 166, 170 and 171 or holes 175, 176, 180, 181, 185 and 186 in FIG 1, depending on shoe size nr, as explained further below. Furthermore, two pins are positioned in the heel region, under the calcaneous bone, illustrated by points 204 and 205 in FIG 2. The positioning of the pins is based on where the highest loads, i.e. pressure, occur on the arch of the foot during walking, in combination with the sensitivity, i.e. nerve distribution, in different parts of the foot. Normally, the front-most pin in the heel region, i.e. pin 116 in FIG 1, gives best feedback, since the fat tissue under this part of the calcaneus bone is relatively thin. The backmost pin in the heel region, i.e. pin 115 in FIG 1, normally gives a first feedback indication that the load limit is imminent. As the gait cycle proceeds and the load is transferred forwardly, the front pins 117, 118 and 119 provide a clear and distinct feedback. The choice of using 5 pins, in combination with their chosen respective position, provides an advantageous solution wherein effective tactile two-point discrimination is obtained. This means that all pins are sensed clearly, distinctly and simultaneously, providing an effective feedback.

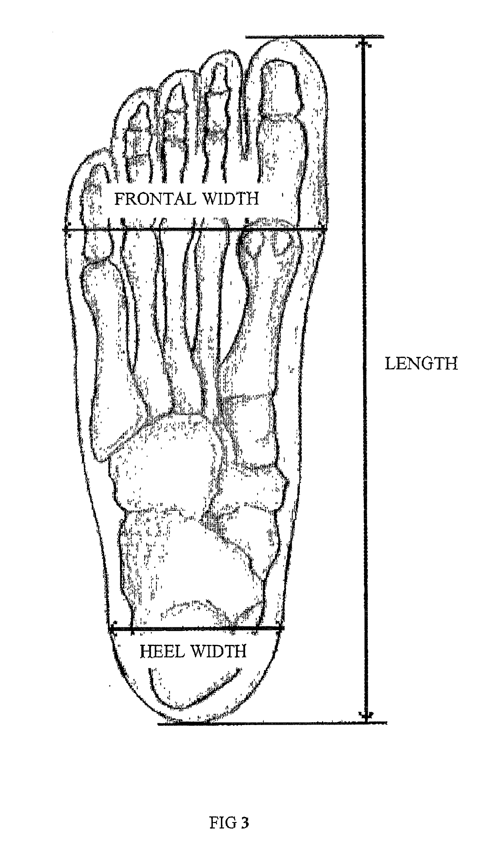

Table 1 illustrates suitable positions for the pins. "Lenght of Foot" in table 1 corresponds to "LENGHT" in FIG 3, "Width of Heel" corresponds to "HEEL WIDTH" in FIG 3, "Width of Frontal Foot Region" in table 1 corresponds with "FRONTAL WIDTH" in FIG 3, "1:st m.h." in table 1 corresponds to length measure 21 in FIG 2, i.e. the length from the back end of the heel to the 1:st metatarsal head, "3:rd m.h." in table 1 corresponds to length measure 23 in FIG 2, i.e. the length from the back end of the heel to the 3:rd metatarsal head, "5:th m.h." in table 1 corresponds to length measure 25 in FIG 2, i.e. the length from the back end of the heel to the 5:th metatarsal head.

**Table 1**

| **Shoe Size nr (SE standard)** | **Lenght of Foot** | **Width of Heel** | **Width of Frontal Foot Region** | **1:st m.h.** | **3:rd m.h.** | **5:th m.h.** |
|---|---|---|---|---|---|---|
| 35 | 22,5 | 5 | 9 | 15,5/3 | 15 | 14/3,5 |
| 35 | 21 | 5 | 9 | 14/3 | 14,5 | 13,5/3 |
| 36 | 22 | 5,5 | 9 | 15/3 | 14,5 | 13/3,5 |
| 36 | 22 | 5,5 | 9 | 14,5/3 | 13,5 | 15/3,5 |
| 37 | 21,5 | 5,5 | 9 | 14,5/3,5 | 15,5 | 14,5/3 |
| 37 | 21 | 5 | 9,5 | 14/3 | 15 | 14/3 |
| 38 | 23,5 | 5 | 9,5 | 14,5/3,5 | 15,5 | 14/3 |
| 38 | 23,5 | 6 | 9,5 | 15/3,5 | 16 | 15/3,5 |
| 39 | 22 | 6 | 9 | 14,5/3,5 | 15 | 13/4 |
| 39 | 22,5 | 6,5 | 9 | 13,5/3 | 14 | 13,5/3,5 |
| 40 | 23 | 6,5 | 9 | 14/3,5 | 15,5 | 13,5/3,5 |
| 40 | 24 | 6 | 9 | 15/4 | 16,5 | 15/3,5 |
| 41 | 24 | 7 | 10 | 17/5,5 | 18,5 | 16,5/3,5 |
| 41 | 25 | 7,5 | 10 | 16,5/4,5 | 18 | 15/4 |
| 42 | 28 | 7,5 | 10 | 19/4 | 19,5 | 18,5/3,5 |
| 42 | 25,5 | 6,5 | 10 | 17/3,5 | 16,5 | 16/4 |
| 43 | 26 | 7 | 10 | 16/3,5 | 17 | 16/3 |
| 43 | 27 | 7,5 | 10 | 17/3 | 17 | 16/4 |
| 44 | 26 | 7 | 10 | 16,5/3,5 | 18 | 17/4 |
| 44 | 26 | 6,5 | 10 | 17/4,5 | 17,5 | 16,5/4 |
| 45 | 27 | 6,5 | 10 | 18/4,5 | 18 | 17/3,5 |
| 45 | 26 | 6,5 | 10 | 17/4,5 | 17,5 | 16,5/4 |
| 46 | 27 | 6,5 | 10 | 18/4,5 | 18 | 17/3,5 |
| 46 | 28 | 7 | 10 | 17,5/4 | 17 | 17/4 |
| 47 | 28,5 | 8 | 10 | 16,5/3,5 | 17 | 16/3,5 |
| 47 | 28,5 | 7,5 | 10 | 17/4,5 | 18 | 17/3,5 |

The various foot positions for the pins, as indicated in table 1, are then translated into suitable positions for the holes 120, 130, 140, 150, 160, 165, 170, 175, 180, 185 of the bottom insole 110 and corresponding matching holes 121, 131, 141, 151, 161, 166, 171, 176, 181, 186 of the top insole 112, in FIG 1.

FIG 4 illustrates a top view of the assembled insole 113, or of the bottom insole 110 or of the top insole 112 of FIG 1, according to one embodiment of the invention. The insole 410, 412, has an outer edge 498, the contour line of which substantially forms an insole with size number 47, according to a Swedish shoe size number standard. A horizontal system of coordinates is associated with the insole 410, 412. The Y-axis of the system of coordinates is parallel with a sagittal plane of a foot during normal operation, i.e. during walking. The Y-axis is substantially directed in a frontal direction of the insole 410, 412, i.e. in a straight forward direction during walking with the insole 410, 412, as illustrated in FIG 4. The X-axis is orthogonal to the Y-axis and is directed in a medial direction, during normal operation of the insole 410, 412. The origin of coordinates is positioned in a backmost edge end 425 of the insole, when being aligned with a foot during normal operation, as illustrated in FIG 4. The X-axis and Y-axis define coordinates of length in mm. The insole 410, 412, has a length 435 of about 299 mm, i.e. the length from a backmost edge end 425 to a foremost edge end 426 is about 299 mm in the Y-direction, when the insole is so aligned, a heel region width 436 of about 70 mm, i.e. it has a length in the medial X-direction of about 70 mm when so aligned, a waist region width of about 65 mm, i.e. it has a length in the medial X-direction of about 65 mm when so aligned, and a frontal region width 437 of about 100 mm, i.e. it has a length in the medial X-direction of about 100 mm when so aligned. According to the invention, the thorough holes 120, 130, 140, 150, 160, 165, 170, 175, 180, 185 of the bottom insole 110 and corresponding matching holes 121, 131, 141, 151, 161, 166, 171, 176, 181, 186 are positioned where they provide relevant tactile feedback. A position too close to the outer edge 498 of the insole 410, 412, does not provide very useful feedback, why there is a minimum distance of 7 mm, and more preferably 12 mm, between an edge of a through hole 120, 130, 140, 150, 160, 165, 170, 175, 180, 185 of the bottom insole 110, or holes 121, 131, 141, 151, 161, 166, 171, 176, 181, 186 of the top insole 112, and the outer edge 498 of the insole 410, 412, according to the invention. More preferably, said through holes are positioned within region/s 452 and/or 453, illustrated in FIG 4.; said regions (452, 453) being defined by the following:
- a first set of coordinates fulfilling the criteria ofy<117 and having a minimum distance to the outer edge of the insole 498 of 12 mm, wherein the first set of coordinates define the first region 452 of the insole, and,
- a second set of coordinate points being defined by fulfilling the criteria of 157,5<y<273 and having a minimum distance to an outer edge of the insole 498 of 12 mm, wherein the second set of coordinates define the second region 453 of the insole. This means that no part of the cross section area of the through holes extend outside region/s 452 and/or 453. The through holes 120, 130, 140, 150, 160, 165, 170, 175, 180, 185 of the bottom insole 110, or holes 121, 131, 141, 151, 161, 166, 171, 176, 181, 186 of the top insole 112 are preferably circular, i.e their respective cross section area is circular in shape, but the invention is not restricted hereto. Circular cylindrical pins 115, 116, 117, 118, 119, are advantageous since they can be produced with high accuracy at low cost, and provide good tactile feedback characteristics. Circular holes are preferably used with circular pins, for obtaining the best tactile feedback characteristics. Furthermore, for obtaining useful tactile feedback characteristics, the foot contact area of the pins, and consequently also the cross section area of the through holes 121, 131, 141, 151, 161, 166, 171, 176, 181, 186, of the top insole, should normally neither be too small, giving rise to risk of penetration through the skin of the foot, nor too large, giving rise to risk of loosing the sensation of distinct tactile feedback of discomfort and in the worst case may give a positive stimulation effect, i.e. the patient obtains an erroneous comfortable tactile sensation when the actual weigh load on the leg/foot is too high for providing optimal healing. The holes 120, 130, 140, 150, 160, 165, 170, 175, 180, 185, of the bottom insole 110 are arranged to provide good snap on and support characteristics for the pins 115, 116, 117, 118, 119, and are therefore preferably circular cylinders having a diameter being less than the diameter of the holes of the top insole and in agreement with the diameter of a waist section of a pin 115 allowing the waist section to be firmly supported by the bottom insole. The diameter of the waist section of the pin 115 must not be too small due to structural strength requirements. Therefore, the cross section area of the through holes 120, 130, 140, 150, 160, 165, 170, 175, 180, 185, 121, 131, 141, 151, 161, 166, 171, 176, 181, 186, is at least 3 mm² and does not exceed 300 mm², according to the invention. Preferably, the through holes 120, 130, 140, 150, 160, 165, 170, 175, 180, 185, 121, 131, 141, 151, 161, 166, 171, 176, 181, 186, are circular cylinders having a diameter in the range of 3-10 mm, and more preferably, a diameter in the range of 3,5-8 mm. In a most preferred embodiment, the diameter of the through holes 121, 131, 141, 151, 161, 166, 171, 176, 181, 186 of the top insole is 5 mm, and exceeds the diameter of the pin heads with 0,5 mm, and the diameter of the through holes 120, 130, 140, 150, 160, 165, 170, 175, 180, 185 of the bottom insole is 4 mm and equals the diameter of a waist section 8220 of the pin 115.

FIG 5 illustrates a top view of the assembled insole 113, or of the bottom insole 110 or of the top insole 112 of FIG 1, illustrating the positioning of the 10 through holes 520, 521; 530, 531; 540, 541; 550, 551; 570, 571; 565, 566; 560, 561; 575, 576; 580, 581 and 585, 586, respectively, according to a preferred embodiment of the present invention. The size and form of the insole of FIG 5 equal the size and form of the insole 410, 412 in FIG 4. A system of coordinates is associated with the insole 510, 512 in FIG 5 in the same manner as described for the insole 410, 412, in FIG 4, which is illustrated by an X- and Y-axis in FIG 5. The insole 510, 512 has 10 through holes 520, 530, 540, 550, 560, 565, 570, 575, 580, 585, or 521, 531, 541, 551, 561, 566, 571, 576, 581, 586, wherein each respective hole is being positioned as follows:
- a first hole (520, 521) positioned at the coordinate point (x=0, y=31),
- a second hole (530, 531) positioned at the coordinate point (x=-0,7, y=46),
- a third hole (540, 541) positioned at the coordinate point (x=-1,2, y=58),
- a fourth hole (550, 551) positioned at the coordinate point (x=-1,7, y=70),
- a fifth hole (560, 561) positioned at the coordinate point (x=-23, y=198),
- a sixth hole (565, 566) positioned at the coordinate point (x=0, y=205),
- a seventh hole (570, 571) positioned at the coordinate point (x=22, y=195),
- an eighth hole (575, 576) positioned at the coordinate point (x=-25, y=210),
- a ninth hole (580, 581) positioned at the coordinate point (x=0, y=220),
- a tenth hole (585, 586) positioned at the coordinate point (x=30, y=208).

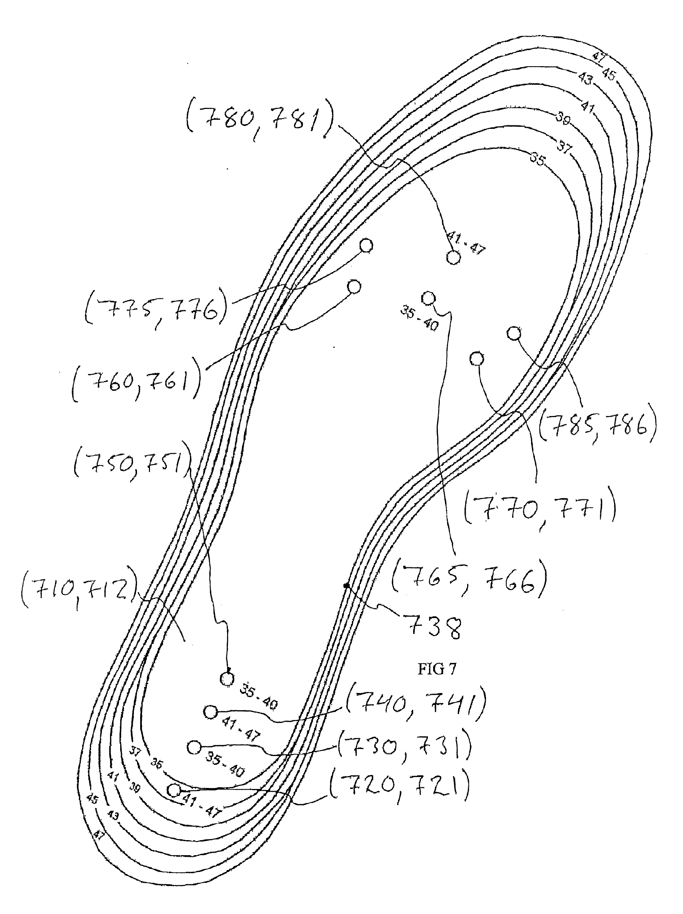

Theoretically, an unlimited number of pins/holes can be used, however using too many pins takes away the tactile feedback characteristics of the insole assembly that the invention seeks to provide. The choice of using exactly 5 pins and 10 holes for the bottom insole 510 and top insole 512 has proven to give a well functioning insole during tests. The choice of providing 10 through holes in the bottom insole 510 and top insole 512, and being positioned as illustrated in FIG 5, gives the advantage that one relatively large insole, e.g. having a Swedish size number 47 according to the mentioned standard, can be used by patients having different shoe size numbers, as described further below with reference to FIG 7. As stated above, the diameter of the 10 through holes 521, 531, 541, 551, 561, 566, 571, 576, 581 and 586 of the top insole 512 is 5 mm, and the diameter of the through holes 520, 530, 540, 550, 560, 565, 570, 575, 580 and 585 is 4 mm, in a most preferred embodiment wherein said through holes matches and functions well with the pin 115, as described further below.

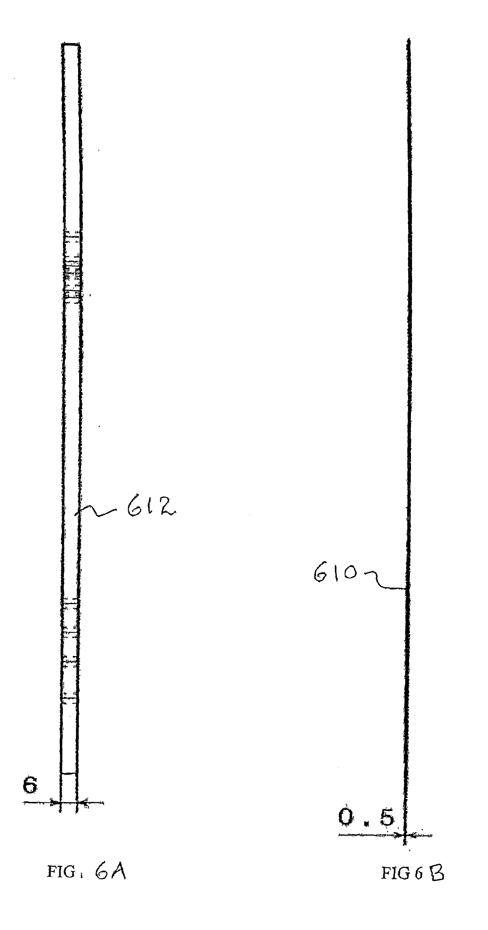

FIG 6A is a side view of the top insole 612. The top insole 612 has preferably a thickness of 6mm at rest, as illustrated in FIG 6A, in order not to become too bulky and provide good compressibility characteristics. According to the invention, the top insole 612 is made of an elastic/compressible material allowing it to compress when being subject of pressure, as when it is squeezed between a foot and the bottom insole 610 during normal operation, as during walking. According to one embodiment of the invention, the top insole 612 compresses about 1-3 mm, at least in central parts of the regions 452, 453, when the top insole 612 is being installed in a shoe and is being squeezed between a foot and the shoe during walking, wherein the weight load on the foot is in the range of about 10-80 Kg. Thus, the thickness of the top insole 612 decreases to about 3-5 mm for a specific threshold pressure during walking, corresponding to a maximum weight load of e.g. 30 Kg on the foot. Furthermore, according to the invention, the material of the top insole 612 normally allows the insole 612 to resume its substantial rest thickness of about 6 mm during a swing phase of the gait cycle, i.e. the phase when the top insole 612 is not squeezed by the foot. Normally, the top insole 612 must resume its substantial rest dimensions at least within about 3 seconds after foot pressure release, and more preferably within 1 second after foot pressure release. Suitable materials for the top insole 612 are therefore cellular plastic materials, e.g. cellular urethanes which are medium density, microcellular foam materials, or cellular rubber materials, having elastic/compressibility/resilience characteristics meeting above functionality requirements. This means that the material of the top insole 612 has a hardness Shore "0" value generally in the range of A 4-30, and preferably in the range of A 5-20, when measured according to the testing specification ASTM D2240, issued by the American Society for Testing and Material, and a vertical resilience rebound value in the range of 4-40 as measured by a Shore instrument Resiliometer, avg (Ball Rebound Tester) according to the testing specification ASTM D 2632-92, issued by the American Society for Testing and Material.

Urathene materials provided by PORON MEDICAL^{®}, which are commercially available e.g. from Rogers Corporation, High Performance Foams Division, Chicago, USA, are suitable for the top insole 612 according to the invention. Table 2 specifies some suitable PORON^{®} materials for the top insole according to a preferred embodiment. Apart from providing well functioning elastic/compressibility/resilience characteristics to the insole 612, these PORON^{®} materials also provide long term comfort and good hygiene, since they "breathe", are fungal resistant, have a relatively low water absorption and have a relatively smooth surface.

**Table 2**

| **Name of Material** | **Reference Code** | **Denisty (kg/m3)** | **Thickness (mm)** | **Shore "0"** | **Water Absorption** | **Colour** |
|---|---|---|---|---|---|---|
| PORON^{®}So ft-Cushioning | PORON^{®}M S-240060-05 | 240±10 | 6 | 12 | <20% | Neutral |
| PORON^{®}So ft-Cushioning | PORON^{®}M S-240060-72 | 240±10 | 6 | 12 | <20% | Aquama rine |
| PORON^{®}So ft-Supporting | PORON^{®}M S-320060-40 | 320±10 | 6 | 17 | <20% | Beige |
| PORON^{®}Fi rm-Energy Absorbing | PORON^{®}M F-240060-11 | 240±10 | 6 | 18 | <20% | Navy |
| PORON^{®}Fi rm-Energy Absorbing | PORON^{®}M F-240060-41 | 240±10 | 6 | 18 | <20% | Panel Tan |
| PORON^{®}EX traSoft- Slow Rebound | PORON^{®}4 701-30-15500-04 | 320±10 | 12,7 | 8 | 9% | Black |

The materials specified on rows 2-4 and 6 in table 2 worked very well during practical tests in the foot weight load range of about 15-55 Kg for a group of test patients. Each PORON^{®}material in table 2 is associated with a specific weight load limit, which is individual and varies to some extent for different patients. The materials indicated on rows 1 and 2 constitute alternatives for substantially the same weight threshold and the materials indicated on rows 4 and 5 constitute alternatives for substantially the same weight threshold. A person skilled in the art realises that other PORON^{®} materials may be used, e.g. for other foot weigh loads. For low foot weight loads, e.g. 10 Kg or less, the top insole 612 may be made of a polyether material.

Table 3 specifies various cellular rubber materials, which the top insole 612 is made of, according to an alternative embodiment. These cellular rubbers are commercially available e.g. from National Gummi AB, Fagerdala Cellplaster, Sweden. Shore "0" and Shore "00" are different hardness scales.

**Table 3**

| **Cellular Rubber Material** | **Density (kg/m³**) | **Thickness (mm)** | **Shore"00" value** | **Water Absorption** | **Colour** |
|---|---|---|---|---|---|
| CR1212 | 205±15 | 5 | 40-50 | <5% | Red-Brownish |
| CR1227 | 155±25 | 5 | 50-70 | <5% | Black |
| EPDM1723 | 125±25 | 5 | 20-40 | <5% | Black |
| EI718 | 175 | 5 | 40 | Not Available | Black |
| ES616 | 120-170 | 5 | 40-60 | 5% | Black |
| NR820 | 192-320 | 5 | 5-9 PSI | 5% | Black |

FIG 6B is a side view of the bottom insole 610 according to one embodiment of the invention. The bottom insole 610 has a thickness of 0,5 mm and is preferably made of a thermoplastic polyamide material, PA 6, giving it good mechanical and supporting characteristics and a relatively low weight. PA 6 is a conventional commercial term for such polyamide material. Table 4 specifies the main mechanical characteristics of the PA 6 material, and a person skilled in the art can find other suitable materials with similar characteristics. An important aspect is to keep the thickness of the insole arrangement as small as possible, thereby making the insole less bulky which facilitates installation in a shoe, and at the same time provide a distinct and precise tactile feedback. The thickness of the bottom insole 610 of 0,5 mm and the top insole 612 of 6 mm at rest constitutes an advantageous combination, according to a preferred embodiment of the invention.

**Table 4**

| **Material** | **Density (kg/m³**) | **Coefficient of elasticity (GPa)** | **Breaking strain (Mpa)** | **Bending Strength %** | **Break Elongation %** | **Water Absorbation%** |
|---|---|---|---|---|---|---|
| PA 6 | 1,13 | 1,4 | 65 | 55 | 200 | 1,8 |

FIG 7 illustrates a top view of an insole 710, 712 according to one embodiment of the invention. There are 6 size number contour lines printed on the top surface of the insole 710, 712, the sizes corresponding to the Swedish shoe size number standard. For instance, the contour line 738 represents size nr 35, as illustrated in FIG 7. This allows a cost effective production since the insole 710, 712 need only be produced in one size, i.e. size 47 in this case. As illustrated in FIG 7, the position of the 10 through holes 720, 721, 730, 731, 740, 741, 750, 751, 760, 761, 770, 771, 775, 776, 780, 781, 785, 786 are chosen so that the functionality of the insole 710, 712 is not compromised for any shoes size.

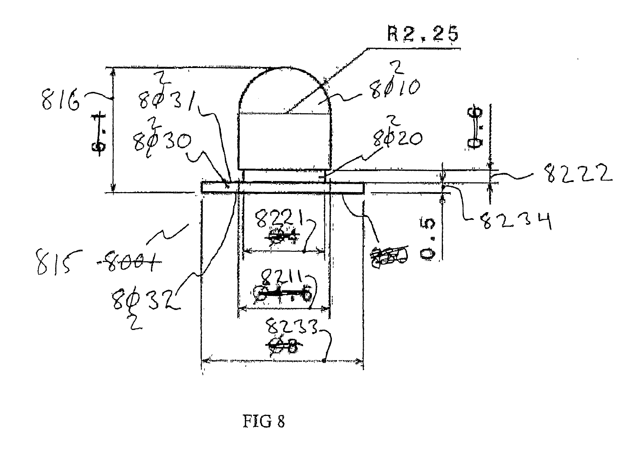

FIG 8 illustrates a tactile feedback element 815 according to one embodiment of the invention wherein the tactile feedback element is realised as a pin element 815. However, it shall be understood that the invention is not restricted to a pin element 815 as shown in FIG 8, but any feedback element providing suitable tactile overload feedback when protruding from the (top) insole may be used, according to the invention. For instance, the tactile feedback element may be realised as a half circular disk or a half sphere or a spike element made of a suitable firm and rigid material, and designed to be fastened/anchored in the (bottom) insole, but many other possibilities exist, obvious to a person skilled in the art. In Fig 8, the pin 815 has a tapered head section 8210, a waist section 8220 being circular cylindrical, and a supporting bottom section 8230.The upper tapered end of the head 8210 section has a half sphere form, and the upper surface 8231 and the bottom surface 8232 of the bottom section 8230 is planar. This provides good tactile stimulation and supporting characteristics for the insole arrangement according to the invention. In FIG 8, the maximum diameter 8211 of the head section 8210 is about 4,5 mm, the total height 816 of the pin is about 6,1 mm, the diameter 8221 of the waist section 8220 is about 4 mm and the height 8222 of the waist section 8220 is about 0,6 mm, the maximum diameter 8233 of the bottom section 8230 is about 8 mm and the height 8234 of the bottom section 8230 is about 0,5 mm. This provides good tactile stimulation and supporting characteristics and makes it easy to snap on/off the pin 815 on/from the bottom insole. The pin 815 may be made of a metallic material, such as automatic screw steel, however other hard materials, such as hard plastic materials, may also be used. A suitable automatic screw steel, SS 1914, for the pin 815 comprises 0,06-012% C, about 0,02% Si, 0,9-1,3% Mn, and about 0, 1 % Pb. Table 5 specifies some physical data for the automatic screw steel, SS 1914.

| Material | Breaking strength Rm (N/mm²) | Elongation limit Rp 0,2 (N/mm²) | Hardness (HB) |
|---|---|---|---|
| SS 1914 | 510-750 | 410 | 170-220 |

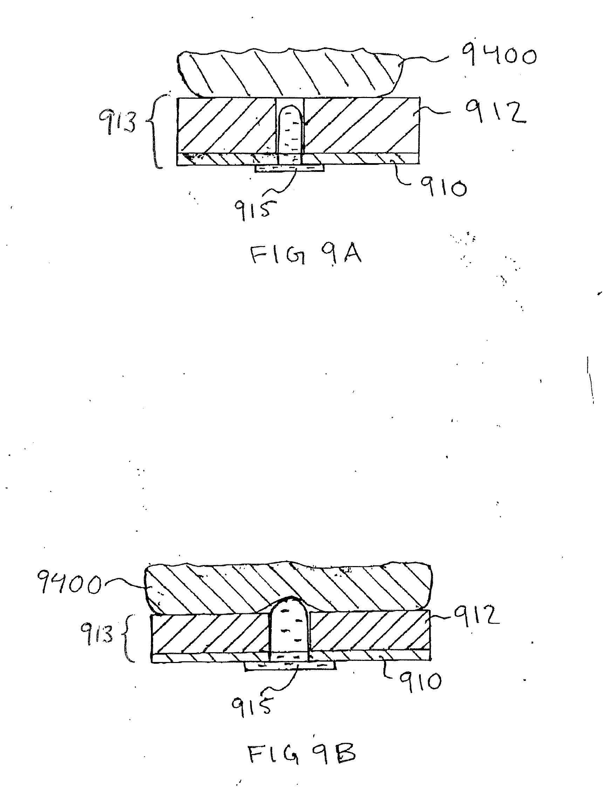

FIG 9A illustrates an assembled insole kit 101 of FIG 1 forming an orthopedic insole 913, when the insole 913 is in a rest condition and not being pressed by a foot 9400. As illustrated in FIG 9A, the tactile overload feedback element 915 does not protrude from the insole 913 when the insole is at rest, i.e. not being compressed, and thus provides no tactile stimulation to the foot 9400 in this rest condition.

FIG 9B illustrates an assembled insole kit 101 of FIG 1 forming an orthopedic insole 913, when the insole 913 is in a loaded condition and being pressed by a foot 9400.

Now, with reference to FIG 1-10, the usage of the insole arrangement according to the present invention will be described.

Assuming that a patient with shoe size number 45 and weighing about 85 Kg needs to stimulate the right foot/knee for optimising the healing process, e.g. after a sprain of the right foot or after a surgical operation of the right knee. A bottom insole and a top insole of size 45 is then first cut out from a bottom insole 710 and a top insole 712. The 5 pins 115, 116, 117, 118 and 119 are then snapped into the bottom insole 710, in through holes 720, 740, 775, 780 and 785 respectively. The top insole is then fastened on top of the bottom insole, e.g. by means of adhesive tape or similar, and the so created insole arrangement is installed in a right shoe, which normally is a shoe of the patient. The patient then puts this shoe on. Thereafter, the patient in an upright normal walking position puts the right foot/shoe on a balance without putting any weight load on the foot. In this position, the right foot 9400 is not in contact with any of the pins, illustrated by the pin 915 in FIG 9A. The person responsible for the rehabilitation activity, normally a physiotherapist, then decides a suitable initial maximum weight load threshold, e.g. 20 Kg in this case. Normally, there is no direct correlation between this weight load threshold and the weight of the patient. Thereafter, the patient carefully starts to put weight load on the right foot, thereby compressing the top insole 912. If the threshold limit of 20 Kg is reached without the patient sensing/feeling any of the pins 915, then the current top insole is replaced with another top insole made of a softer material. For instance, if the initial top insole 912 is made of PORON^{®}MS-240060-05 and having a Shore "0" hardness value of 12, then this top insole 912 is replaced by a top insole 912 made of e.g. PORON^{®}4701-30-15500-04 having a Shore "0" hardness value of 8. This procedure is repeated until the patient feels any of the pins 915 within the weight load limit of 20 Kg, meaning that the pin 915 is in contact with the foot 9400, as illustrated in FIG 9B. At this point, the pin 915 will cause pain if the patient further increases the load, thereby providing an effective auto overload control mechanism. The patient then starts the rehabilitation by walking on crutches (or using another suitable support), without risk of overload. The top insole 915 is replaced with a new harder top insole, i.e. having a higher Shore "0" value, successively during the healing process, e.g. every 2 weeks.

The insole according to the invention can be used for both left and right feet, i.e. there is no need to produce "left" and "right" variants. The bottom insole and pins may be washed/sterilised and reused, even by a plurality of patients. The top insole is normally discarded after being used due to hygienic reasons.

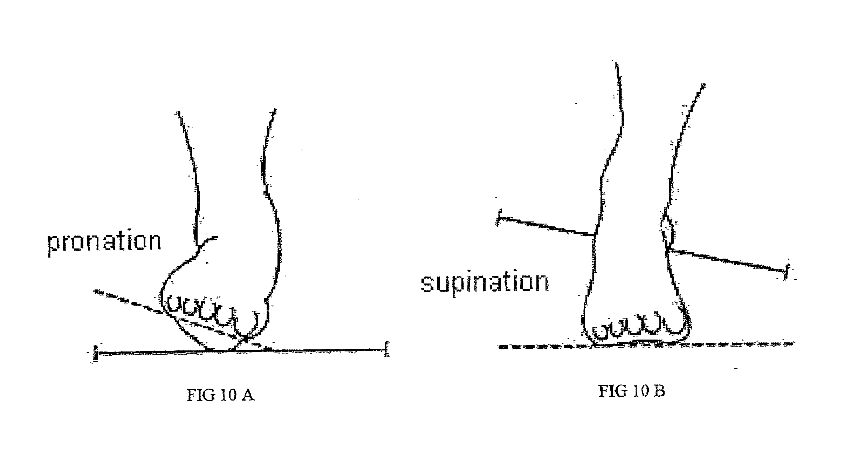

The insole arrangement according to the invention may be used by a wide range of patient groups, wherein some minor modifications may be necessary, e.g. it may be used in a plaster, it may be used by patients who tend to put a too high lateral or medial load on the foot, i.e. suffering from pronation or supination as illustrated in FIG 10 A and B, or by patients who tend to put a too high frontal load on the foot, referred to as "toe walkers". In these cases it may be necessary to position the holes (120, 121, 130, 131, 140, 141, 150, 151, 160, 161, 165, 166, 170, 171, 175, 176, 180, 181, 185, 186) closer to the outer edge of the insole than what is described for the preferred embodiments above. A person skilled in the art realises how to modify the preferred embodiments discussed above in order to exploit the invention also for/in these patient groups/cases.

The described insole arrangement according to the invention provides accurate tactile feedback allowing an efficient auto load control of the lower extremities during rehabilitation, and thereby an improved healing and a shortened rehabilitation period. The insole arrangement is versatile and cost effective, both to produce and to use. It is user friendly and intuitive in its use. It may be used without notice, which may be of importance to some patient groups. as a person skilled in the art understands.

The principles of the present invention have been described in the foregoing by means of examples and/or embodiments and/or modes/examples of operation. However, as already stated, many modifications and/or combinations are possible, e.g. regarding the choice of material and the absolute and relative dimensions of different parts of the insole arrangement described above. Furthermore, the insole according to the invention need not necessarily be formed by separate top/bottom insoles as described above, but may instead have a completely integrated design with the bottom/top insole designed as one integrated insole, and with integrated pins etc. Therefore, the invention should not be construed as being limited to the particular embodiments/working examples discussed above, and it should be appreciated that variations may be made in those embodiments/working examples by persons skilled in the art, without departing from the scope of the present invention as defined by the appended claims.

## Claims

1. A compressible insole (913) comprising at least one hole (121, 131, 141, 151, 161, 166, 171, 176, 181, 186), **characterised in that** it comprises at least one tactile overload feedback element (115, 116, 117, 118, 119) being anchored in the insole and being arranged to slide inside the hole (121, 131, 141, 151, 161, 166, 171, 176, 181, 186) in a substantially perpendicular direction relative a foot during normal operation when the insole (913) is being compressed at weight bearing, as during walking, thereby protruding from the insole (913) at some compression of the insole (913).

2. The insole (913) according to claim 1 further **characterised in that** the hole (121, 131, 141, 151, 161, 166, 171, 176, 181, 186) is positioned vertically under the forefoot, or the heel of the foot, during normal operation, as during walking.

3. The insole (913) according to claim 2 further **characterised in that** the hole (121, 131, 141, 151, 161, 166, 171, 176, 181, 186) is positioned under a first metatarsal head of the foot or under the third metatarsal head of the foot or under the fifth metatarsal head of the foot or under the calcaneus bone of the foot during normal operation, as during walking.

4. The insole (913) according to claim 3 further **characterised in that** it comprises five holes (121, 141, 176, 181, 186) perpendicularly oriented relative the foot, and five tactile overload feedback elements (115, 116, 117, 118, 119), each tactile overload feedback element (115, 116, 117, 118, 119) being arranged to slide in one of the respective holes (121, 141, 176, 181, 186) during weight bearing, as during walking, wherein two of the holes (121, 141) are positioned under the calcaneus bone, one hole (186) is positioned under the first metatarsal head, one hole (181) is positioned under the third metatarsal head and one hole (176) is positioned under the fifth metatarsal head during normal operation, as during walking.

5. The insole (913) according to any of claims 1-4 further **characterised in that** the insole (913) comprises a bottom insole (110) and a top insole (112) arranged on top of the bottom insole (110) wherein the bottom insole (110) is made of a firm material, such as a thermoplastic polyamide material, and said tactile overload feedback element is anchored in said bottom insole (110); and wherein said top insole (112) is made of a compressible material, such as a cellular plastic material, a cellular urethane material, a polyether material or a cellular rubber material, and comprises said at least one hole (121, 131, 141, 151, 161, 166, 171, 176, 181, 186) in which the tactile overload feedback element is arranged to slide during normal operation.

6. The insole (913) according to claim 5 further **characterised in that** the bottom insole (110) comprises at least one through hole (120, 130, 140, 150, 160, 165, 170, 175, 180, 185) matching said at least one through hole (121, 131, 141, 151, 161, 166, 171, 176, 181, 186) of the top insole (112) during normal operation, wherein the at least one tactile overload feedback element (115) is snapped onto the bottom insole (110) through said through hole (120, 130, 140, 150, 160, 165, 170, 175, 180, 185) of the bottom insole (110).

7. The insole (913) according to claim 5 or 6 further **characterised in that** the top insole (112) is made of a compressible material having a hardness shore "0" value in the range of A 4-30.

8. The insole (913) according to claim 5, 6 or 7 wherein the insole (913), when being aligned with a sagittal plane of a foot during normal operation, and further being associated with an imaginary system of coordinates defining a first and second region (452, 453) of the insole (913); said system of coordinates having an Y-axis being parallel with said sagittal plane and substantially directed in a frontal direction of the insole (913), and having an X-axis directed in a medial direction, and having its origin of coordinates positioned at the backmost edge end (425) of the insole (913) when so aligned; the X-axis and Y-axis defining coordinates of length in mm; said regions (452, 453) being defined by the following:
- a first set of coordinates fulfilling the criteria of y<117 and having a minimum distance to an outer edge of the insole (498) of 12 mm, wherein the first set of coordinates define the first region (452) of the insole, and,
- a second set of coordinate points being defined by fulfilling the criteria of 157,5<y<273 and having a minimum distance to an outer edge of the insole (498) of 12 mm, wherein the second set of coordinates define the second region (453) of the insole,
wherein the insole (913) have a total length (435) of about 299 mm from the back edge end (425) to a front edge end (426), a heel region width (436) of about 70 mm and a front foot region width (437) of about 100 mm, further being **characterised**
**in that** the at least one through hole (420, 430, 440, 450, 460, 465, 470, 475, 480, 485) of the bottom insole (410) and the at least one through hole (421, 431, 441, 451, 461, 466, 471, 476, 481, 486) of the top insole (412) lead to said first region (452) or to said second region (453) of the insole (913).

9. The insole (913) according to any of claim 8 further **characterised in that** the top insole (512) and the bottom insole (510) each comprise 10 respective matching through holes (521, 531, 541, 551, 561, 566, 571, 576, 581, 586 and 520, 530, 540, 550, 560, 565, 570, 575, 580, 585, respectively), each respective through hole being positioned as follows:
- a first respective through hole (520, 521) positioned at the coordinate point (x=0, y=31),
- a second respective through hole (530, 531) positioned at the coordinate point (x=-0,7, y=46),
- a third respective through hole (540, 541) positioned at the coordinate point (x=-1,2, y=58),
- a fourth respective through hole (550, 551) positioned at the coordinate point (x=-1,7, y=70),
- a fifth respective through hole (560, 561) positioned at the coordinate point (x=-23, y=198),
- a sixth respective through hole (565, 566) positioned at the coordinate point (x=0, y=205),
- a seventh respective through hole (570, 571) positioned at the coordinate point (x=22, y=195),
- an eighth respective through hole (575, 576) positioned at the coordinate point (x=-25, y=210),
- a ninth respective through hole (580, 581) positioned at the coordinate point (x=0, y=220),
- a tenth respective through hole (585, 586) positioned at the coordinate point (x=30, y=208), and wherein five tactile overload feedback elements (115, 116, 117, 118, 119) are being arranged to slide vertically in a respective hole of the top insole (512) during normal operation.

10. The insole (913) according to any of claims 5-9 **characterised in that** the top insole (612) has a thickness of about 6 mm at rest and at least in some region is compressed at least about 1 mm during normal operation, as during walking.

11. The insole (913) according to any of claims 5-10 **characterised in that** the top insole (612) resumes its substantial rest thickness of about 6 mm within about 3 seconds after foot pressure release.

12. The insole (913) according to any of claims 5-11 **characterised in that** the bottom insole (510) has a thickness of about 0,5 mm and wherein the diameter of the at least one through hole (530, 540, 550, 560, 565, 570, 575, 580, 585) of the bottom insole (510) is about 4 mm and the diameter of the at least one through hole (521, 531, 541, 551, 561, 566, 571, 576, 581, 586) of the top insole (512) is about 5 mm.

13. The insole (913) according to any of claims 1-12 further **characterised in that** said tactile overload feedback element (115) is made of a firm material, such as plastic or a metallic material, and is realised as a pin element (815) or a half disk element or a half spherical element.

14. A bottom insole (110) **characterised in that** it is a bottom insole (110) as defined according to any of claims 5-12.

15. The bottom insole (110) according to claim 14 **characterised in that** it has at least one shoe size number contour line (738) printed or otherwise outlined on it which contour line (738) corresponds to a shoe number size in the size number range of 35-47 according to a Swedish shoe size standard.

16. A top insole (112) **characterised in that** it is a top insole (112) as defined according to any of claims 5-12.

17. The top insole (112) according to claim 16 **characterised in that** it has at least one shoe size number contour line (738) printed or otherwise outlined on it which contour line (738) corresponds to a shoe number size in the size number range of 35-47 according to a Swedish shoe size standard.

18. An insole kit **characterised in that** it comprises a bottom insole (110) according to claim 14 or 15 and a top insole (112) according to claim 16 or 17.

19. The kit according to claim 18 further comprising a tactile overload feedback element (815) according to claim 13, wherein the tactile overload feedback element (815), when installed in the insole (913), penetrates about 5 mm into the at least one through hole (121, 131, 141, 151, 161, 166, 171, 176, 181, 186) of the top insole (112).
